# EUROPÄISCHE PATENTSCHRIFT

(11) **EP 2 451 770 B1**
(45) Veröffentlichungstag und Bekanntmachung des Hinweises auf die Patenterteilung: **07.01.2015**
(21) Anmeldenummer: 10731732.3
(22) Anmeldetag: 07.07.2010
(51) Int. Cl.: C25B 3/00, C07C 209/02

(54) **VERFAHREN ZUR DIREKTAMINIERUNG VON KOHLENWASSERSTOFFEN ZU AMINOKOHLENWASSERSTOFFEN MIT ELEKTROCHEMISCHER ABTRENNUNG VON WASSERSTOFF UND ELEKTROCHEMISCHER UMSETZUNG DES WASSERSTOFFS ZU WASSER**
PROCESS FOR DIRECT AMINATION OF HYDROCARBONS TO AMINO HYDROCARBONS WITH ELECTROCHEMICAL SEPARATION OF HYDROGEN AND ELECTROCHEMICAL REACTION OF THE HYDROGEN TO WATER
PROCÉDÉ D'AMINATION DIRECTE D'HYDROCARBURES EN AMINO-HYDROCARBURES AVEC SÉPARATION ÉLECTROCHIMIQUE D'HYDROGÈNE ET TRANSFORMATION ÉLECTROCHIMIQUE DE L'HYDROGÈNE EN EAU

(30) Priorität: 10.07.2009 EP 09165224
(43) Veröffentlichungstag der Anmeldung: 16.05.2012
(73) Patentinhaber: BASF SE, 67056 Ludwigshafen (DE)
(72) Erfinder: KUBANEK, Petr, 68163 Mannheim (DE); PANCHENKO, Alexander, 67063 Ludwigshafen (DE); FISCHER, Andreas, 64646 Heppenheim (DE); HEIDEMANN, Thomas, 68519 Viernheim (DE)
(86) Internationale Anmeldenummer: PCT/EP2010/059710
(87) Internationale Veröffentlichungsnummer: WO 2011/003933

(56) Entgegenhaltungen:
- WO-A1-2007/025882
- GB-A- 738 515

## Beschreibung

Die vorliegende Erfindung betrifft ein Verfahren zur Direktaminierung von Kohlenwasserstoffen mit einem Aminierungsreagenz zu Aminokohlenwasserstoffen in Gegenwart eines Katalysators, wobei mindestens ein Teil des bei der Umsetzung entstehenden Wasserstoffs mittels einer gasdichten Membran-Elektroden-Assembly elektrochemisch abgetrennt und der Wasserstoff dabei unter Erzeugung von elektrischem Strom mit Sauerstoff zu Wasser umgesetzt wird.

Die kommerzielle Herstellung von Aminokohlenwasserstoffen aus Kohlenwasserstoffen wird üblicherweise in mehrstufigen Reaktionen durchgeführt. So wird bei der Herstellung von Anilin aus Benzol zunächst ein Benzolderivat wie Nitrobenzol, Chlorbenzol oder Phenol hergestellt, welches anschließend in ein- oder mehrstufigen Reaktionen zu Anilin umgesetzt wird.

Es sind auch Verfahren zur direkten Herstellung von Aminokohlenwasserstoffen aus den entsprechenden Kohlenwasserstoffen bekannt. Reaktionen, bei denen Aminokohlenwasserstoffe direkt aus den entsprechenden Kohlenwasserstoffen hergestellt werden bezeichnet man als Direktaminierung. Wibaut beschreibt 1917 erstmals die heterogenkatalysierte Direktaminierung von Benzol (Berichte 1917, 50, 541-546).

Bei der Direktaminierung findet die Umsetzung des eingesetzten Kohlenwasserstoffs zum entsprechenden Aminokohlenwasserstoff unter Freisetzung von Wasserstoff statt (WO 2007/025882). Bei der Direktaminierung von Benzol zu Anilin entstehen aus 1 mol Benzol und 1 mol Ammoniak 1 mol Anilin und 1 mol Wasserstoff. Die Direktaminierung von Benzol zu Anilin ist durch die Lage des thermodynamischen Gleichgewichts limitiert. Der Gleichgewichtsumsatz liegt bei Temperaturen von 350 °C bei ca. 0,5 Mol-% bezogen auf Benzol.

Aufgrund des niedrigen Gleichgewichtsumsatzes ist zur wirtschaftlichen Durchführung der Direktaminierung eine Verschiebung der Lage des thermodynamischen Gleichgewichts auf die Seite der Aminokohlenwasserstoffe notwendig.

Eine Möglichkeit besteht darin, aus dem Reaktionsgemisch der Direktaminierung, welches Aminokohlenwasserstoff, nicht umgesetzte Edukte und Wasserstoff enthält, einen Teil des Wasserstoffs abzutrennen und die nicht umgesetzten Edukte erneut in die Direktaminierungsreaktion einzusetzten. Die Abtrennung des Wasserstoffs aus dem Reaktionsgemisch ist notwendig, da sonst durch den Wasserstoff das thermodynamische Gleichgewicht in Richtung der Edukte verschoben wird, wodurch die Ausbeute an Aminokohlenwasserstoff bei der erneuten Direktaminierung noch niedriger ausfällt. Eine weitere Möglichkeit besteht darin, den bei der Direktaminierung entstehenden Wasserstoff direkt aus der Reaktionszone zu entfernen. Zur Entfernung des Wasserstoffs aus dem Reaktionsgemisch sind mehrere Verfahren beschrieben.

WO 2007/096297 und WO 2000/69804 beschreiben ein Verfahren zur Direktaminierung von aromatischen Kohlenwasserstoffen zu den entsprechenden Aminokohlenwasserstoffen, wobei der entstehende Wasserstoff durch Oxidation an reduzierbaren Metalloxiden aus dem Reaktionsgemisch entfernt wird. Diese Verfahren haben den Nachteil, dass die reduzierbaren Metalloxide nach einiger Zeit wieder mit Sauerstoff regeneriert werden müssen. Dies bedeutet kostenintensive Unterbrechungen des Verfahrens, da die Direktaminierung der Kohlenwasserstoffe und die Regenerierung der reduzierbaren Metalloxide üblicherweise nicht bei den gleichen Bedingungen ablaufen. Zur Regenerierung des Katalysators muss der Reaktor daher üblicherweise entspannt, gespült und inertisiert werden.

Eine weiter unerwünschte Nebenreaktion, die bei der Direktaminierung von Kohlenwasserstoffen zu Aminokohlenwasserstoffen auftritt, ist die Zersetzung von Ammoniak zu Wasserstoff. Diese Zersetzung ist nachteilig, da einerseits das Edukt Ammoniak verloren geht und andererseits der bei der Zersetzung entstehende Wasserstoff zu einer weiteren ungünstigen Verschiebung der Gleichgewichtslage in Richtung der Edukte führt. Bei den in WO 2007/096297 und WO 2000/69804 beschriebenen Katalysatoren nimmt die unerwünschte Zersetzung von Ammoniak mit steigendem Reduktionsgrad der Metalloxide zu, so dass mit steigendem Reduktionsgrad die Gleichgewichtslage immer weiter in Richtung der Edukte verschoben wird.

WO 2007/099028 beschreibt ein Direktaminierungsverfahren von aromatischen Kohlenwasserstoffen zu den entsprechenden Aminokohlenwasserstoffen, wobei in einem ersten Schritt die heterogenkatalysierte Direktaminierung durchgeführt wird und in einem zweiten Schritt der im ersten Schritt entstandene Wasserstoff durch die Umsetzung mit einem Oxidationsmittel wie Luft, Sauerstoff, CO, CO₂, NO und/oder N₂O umgesetzt wird. Die Verwendung von Oxidationsmitteln wie Sauerstoff führt zur Oxidation von Ammoniak und zur Bildung weiterer Nebenprodukte. Dies führt zu höheren Stoffkosten und zu zusätzlichen Aufarbeitungsschritten, wodurch die Wirtschaftlichkeit des Verfahrens verschlechtert wird.

WO 2008/009668 beschreibt ebenfalls ein Verfahren zur Direktaminierung von aromatischen Kohlenwasserstoffen. Die Entfernung des Wasserstoffs aus dem Reaktionsgemisch wird hier dadurch erreicht, dass die Direktaminierung unter Zusatz von Verbindungen durchgeführt wird, die mit dem bei der Direktaminierung entstehenden Wasserstoff reagieren. Als der Direktaminierung zugesetzte Verbindungen werden beispielsweise Nitrobenzol und Kohlenmonoxid beschrieben. Auch bei diesem Verfahren treten die vorstehend beschriebenen Nachteile auf.

WO 2007/025882 beschreibt die Direktaminierung von aromatischen Kohlenwasserstoffen zu den entsprechenden Aminokohlenwasserstoffen, wobei Wasserstoff aus dem Reaktionsgemisch physikalisch abgetrennt wird. Die Abtrennung erfolgt hier durch eine selektiv wasserstoffdurchlässige Membran, das heißt Wasserstoff wandert als H₂-Molekül durch die Membran. Als Membranmaterialien werden bevorzugt Palladium und Palladiumlegierungen eingesetzt. Die Diffusionsgeschwindigkeit hängt bei diesem Verfahren vom Partialdruckunterschied des Wasserstoffs zwischen der Retentat- und Permeat-Seite der Membran ab. Um höhere Diffusionsgeschwindigkeiten zu erreichen, muss bei höheren Druckunterschieden gearbeitet werden, die hohe Anforderungen an die mechanische Stabilität der Membran stellen. In Basile, Top. Catal. (2008), 51, 107-122, wird zudem beschrieben, dass zum Erreichen einer ausreichend hohen Diffusionsgeschwindigkeit Temperaturen oberhalb 300 °C erforderlich sind. Darüber hinaus müssen zur Ausbildung der Druckunterschiede entsprechende Vorrichtungen zum Verdichten und Expandieren des Gasgemisches vorhanden sein. Aus thermodynamischen Gründen bleibt zudem stets ein gewisser Anteil des Wasserstoffs in dem Retentat zurück. Dies wirkt sich negativ auf die Lage des thermodynamischen Gleichgewichts aus.

Aufgabe der vorliegenden Erfindung ist es daher, ein Verfahren zur Direktaminierung von Kohlenwasserstoffen zu Aminokohlenwasserstoffen bereitzustellen, bei dem Wasserstoff aus dem Reaktionsgemisch möglichst effektiv abgetrennt wird, und dass die vorstehend genannten Nachteile aus dem Stand der Technik bekannten Verfahren zur Direktaminierung vermeidet. Darüber hinaus soll das Verfahren eine Verschiebung der Lage des thermodynamischen Gleichgewichts auf die Seite der Aminokohlenwasserstoffe ermöglichen. Insbesondere soll die Abtrennung des Wasserstoffs direkt aus der Reaktionszone ermöglicht werden. Die eingesetzten Kohlenwasserstoffe sollen ebenso wie die bei der Umsetzung anfallenden Nebenprodukte effizient genutzt werden. Das Verfahren soll eine möglichst günstige Energiebilanz und einen möglichst geringen apparativen Aufwand aufweisen.

Die Aufgabe wird erfindungsgemäß gelöst durch ein Verfahren zur Direktaminierung von Kohlenwasserstoffen zu Aminokohlenwasserstoffen durch Umsetzen eines Eduktstroms E, der mindestens einen Kohlenwasserstoff und mindestens ein Aminierungsreagenz enthält, zu einem Reaktionsgemisch R, enthaltend Aminokohlenwasserstoff und Wasserstoff in einer Reaktionszone RZ, und elektrochemische Abtrennung mindestens eines Teils des bei der Umsetzung entstandenen Wasserstoffs aus dem Reaktionsgemisch R mittels einer gasdichten Membran-Elektroden-Assembly, die mindestens eine selektiv protonenleitende Membran und auf jeder Seite der Membran mindestens einen Elektrodenkatalysator aufweist, wobei auf der Retentatseite der Membran mindestens ein Teil des Wasserstoffs an dem Anodenkatalysator zu Protonen oxidiert wird und die Protonen nach Durchqueren der Membran auf der Permeatseite an dem Kathodenkatalysator mit Sauerstoff zu Wasser umgesetzt werden, wobei der Sauerstoff aus einem Sauerstoff enthaltenden Strom O stammt, der mit der Permeatseite der Membran in Kontakt gebracht wird.

Gegenüber den bekannten Verfahren, bei denen der Wasserstoff durch reduzierbare Metalloxide entfernt wird, weist das erfindungsgemäße Verfahren den Vorteil auf, dass aufwändige und kostenintensive Unterbrechungen des Verfahrens zur Direktaminierung vermieden werden und dass das Verfahren über einen längeren Zeitraum kontinuierlich betrieben werden kann. Das erfindungsgemäße Verfahren kommt zudem im Vergleich zu bekannten Verfahren bei denen gasförmige Oxidationsmittel wie Luft, Sauerstoff, CO, CO₂, NO oder N₂O oder Verbindungen wie Nitrobenzol eingesetzt werden ohne eine apparativ aufwändige und kostenintensive Abtrennung der durch die Zugabe der Oxidationsmittel entstandenen Nebenprodukte aus.

Der besondere Vorteil des erfindungsgemäßen Verfahrens ist die elektrochemische Abtrennung des gebildeten Wasserstoffs aus dem Reaktionsgemisch R mit der gleichzeitigen Gewinnung von elektrischem Strom.

Der Wasserstoff wird dabei nicht, wie aus dem Stand der Technik bekannt, zunächst abgetrennt und anschließend als Wasserstoff einem Strom erzeugenden Verfahren wie einer externen Brennstoffzelle oder Gasturbine zugeführt, sondern die Stromerzeugung erfolgt schon bei der Abtrennung. Im Vergleich zu den aus dem Stand der Technik bekannten Verfahren wird, je nach Blickwinkel, eine Trennvorrichtung oder eine Einheit zur Energieerzeugung aus dem entstandenen Wasserstoff und die damit einhergehenden Energie- und Substanzverluste eingespart.

Das erfindungsgemäße Verfahren stellt somit eine wirtschaftliche Nutzung der eingesetzten Edukte unter gleichzeitiger Produktion von wertvollen Aminokohlenwasserstoffen und elektrischer Energie bereit.

Die Triebkraft der elektrochemischen Wasserstoffabtrennung ist die Reduktion des Sauerstoffs. Da die Abtrennung nicht, wie bei den üblich verwendeten wasserstoffselektiven Membranen, vom Partialdruckunterschied zwischen den beiden Seiten der Membran abhängig ist, kann die Wasserstoffabtrennung bei sehr viel niedrigeren Drücken und Druckunterschieden durchgeführt werden, wobei vorzugsweise auf einen von außen aufgeprägten Druckunterschied völlig verzichtet wird und insbesondere auf Permeat- und Retentatseite der gleiche Druck herrscht. Damit verringert sich die mechanische Beanspruchung der Membran deutlich, was unter anderem ihre Langzeitstabilität erhöht sowie die Auswahl an für die Membran in Frage kommenden Materialien vergrößert. Dies bietet weiterhin die Möglichkeit, die Abtrennung des Wasserstoffs bei niedrigeren Drücken durchzuführen als bei herkömmlichen Membranen.

Die elektrochemische Abtrennung des Wasserstoffs ist im Vergleich zur Abtrennung mittels herkömmlicher wasserstoffselektiver Membranen deutlich effektiver. Bei gleicher Trennleistung kann daher die erforderliche Membranfläche verkleinert werden oder bei gleich bleibender Membranfläche deutlich mehr Wasserstoff abgetrennt werden. Insgesamt ist daher das erfindungsgemäße Verfahren mit einem geringeren apparativen Aufwand verbunden.

Durch die effektivere Wasserstoffabtrennung ist der Anteil des im Reaktionsgemisch verbleibenden Wasserstoffs im Vergleich zu herkömmlichen Verfahren deutlich geringer. Hierdurch wird gegenüber im Stand der Technik beschriebenen Verfahren eine deutlich stärkere Verschiebung des thermodynamischen Gleichgewichts auf die Seite der Aminokohlenwasserstoffe gewährleistet und die Wirtschaftlichkeit der Direktaminierung deutlich verbessert.

Im Folgenden wird die Erfindung ausführlich beschrieben.

### Kohlenwasserstoffe:

Erfindungsgemäß enthält der Eduktstrom E mindestens einen Kohlenwasserstoff. Geeignete Kohlenwasserstoffe, die in das erfindungsgemäße Verfahren eingesetzt werden können, sind beispielsweise Kohlenwasserstoffe, wie aromatische Kohlenwasserstoffe, aliphatische Kohlenwasserstoffe und cycloaliphatische Kohlenwasserstoffe, die beliebig substituiert sein können und innerhalb ihrer Kette bzw. ihres Rings/ihrer Ringe Heteroatome und Doppel- oder Dreifachbindungen aufweisen können. Bevorzugt werden in dem erfindungsgemäßen Aminierungsverfahren aromatische Kohlenwasserstoffe und heteroaromatische Kohlenwasserstoffe eingesetzt.

Geeignete aromatische Kohlenwasserstoffe sind beispielsweise ungesättigte cyclische Kohlenwasserstoffe, die einen oder mehrere Ringe aufweisen und ausschließlich aromatische C-H-Bindungen enthalten. Bevorzugte aromatische Kohlenwasserstoffe weisen einen oder mehrere 5- und/oder 6-gliedrige Ringe auf.

Unter einem heteroaromatischen Kohlenwasserstoff sind solche aromatischen Kohlenwasserstoffe zu verstehen, in denen einer oder mehrere der Kohlenstoffatome des aromatischen Rings durch ein Heteroatom ausgewählt aus N, O und S ersetzt ist/sind.

Die aromatischen Kohlenwasserstoffe bzw. die heteroaromatischen Kohlenwasserstoffe können substituiert oder unsubstituiert sein. Unter einem substituierten aromatischen bzw. heteroaromatischen Kohlenwasserstoff sind Verbindungen zu verstehen, in denen ein oder mehrere Wasserstoffatome, die an ein Kohlenstoff- und/oder Heteroatom des aro-matischen Rings gebunden sind/ist, durch einen anderen Rest ausgetauscht ist/sind. Geeignete Reste sind beispielsweise substituierte oder unsubstituierte Alkyl-, Alkenyl-, Alkinyl-, Heteroalkyl-, Heteroalkenyl-, Heteroalkinyl-, Cycloalkyl- und/oder Cycloalkinylreste; Halogen, Hydroxy, Alkoxy, Aryloxy, Amino, Amido, Thio und Phosphino. Bevorzugte Reste der aromatischen bzw. heteroaromatischen Kohlenwasserstoffe sind ausgewählt aus C₁₋₆-Alkyl, C₁₋₆-Alkenyl, C₁₋₆-Alkinyl, C₃₋₈-Cycloalkyl, C₃₋₈-Cycloalkenyl, Alkoxy, Aryloxy, Amino und Amido, wobei sich die Angabe C₁₋₆ auf die Anzahl der Kohlenstoffatome in der Hauptkette des Alkylrests, des Alkenylrests oder des Alkinylrests bezieht und die Bezeichnung C₃₋₈ auf die Anzahl der Kohlenstoffatome des Cycloalkyl- bzw. Cycloalkenylrings. Es ist weiterhin möglich, dass die Substituenten (Reste) des substituierten aromatischen oder heteroaromatischen Kohlenwasserstoffs ihrerseits weitere Substituenten aufweisen.

Die Zahl der Substituenten (Reste) des aromatischen bzw. heteroraromatischen Kohlenwasserstoffs ist beliebig. In einer bevorzugten Ausführungsform weist der aromatische bzw. heteroaromatische Kohlenwasserstoff jedoch mindestens ein Wasserstoffatom auf, das direkt an ein Kohlenstoffatom oder ein Heteroatom des aromatischen oder heteroaromatischen Rings gebunden ist. Somit weist ein 6-gliedriger Ring bevorzugt 5 oder weniger Substituenten (Reste) auf und ein 5-gliedriger Ring bevorzugt 4 oder weniger Substituenten (Reste). Besonders bevorzugt trägt ein 6-gliedriger aromatischer bzw. heteroaromatischer Ring 4 oder weniger Substituenten, ganz besonders bevorzugt 3 oder weniger Substituenten (Reste). Ein 5-gliedriger aromatischer oder heteroaromatischer Ring trägt bevorzugt 3 oder weniger Substituenten (Reste), besonders bevorzugt 2 oder weniger Substituenten (Reste).

In einer besonders bevorzugten Ausführungsform des erfindungsgemäßen Verfahrens wird ein aromatischer bzw. heteroaromatischer Kohlenwasserstoff der allgemeinen Formel

(*A*)-(*B*)*ₙ*

eingesetzt, worin die Symbole die folgende Bedeutung haben:
- A: unabhängig Aryl oder Heteroaryl, bevorzugt ist A ausgewählt aus Phenyl, Diphenyl, Benzyl, Dibenzyl, Naphthyl, Anthracen, Pyridyl und Chinolin;
- n: eine Zahl von 0 bis 5, bevorzugt 0 bis 4, insbesondere in dem Fall, wenn A ein 6-gliedriger Aryl- oder Heteroaryl-Ring ist; für den Fall, dass A ein 5-gliedriger Aryl- oder Heteroaryl-Ring ist, ist n bevorzugt 0 bis 4; unabhängig von der Ringgröße ist n besonders bevorzugt 0 bis 3, ganz besonders bevorzugt 0 bis 2 und insbesondere 0 bis 1; dabei tragen die übrigen, keine Substituenten B tragenden Kohlenwasserstoff- oder Heteroatome von A Wasserstoffatome oder gegebenenfalls keinen Substituenten;
- B: ist unabhängig voneinander ausgewählt aus der Gruppe bestehend aus Alkyl, Alkenyl, Alkinyl, substituiertem Alkyl, substituiertem Alkenyl, substituiertem Alkinyl, Heteroalkyl, substiuiertem Heteroalkyl, Heteroalkenyl, substituiertem Heteroalkenyl, Heteroalkinyl, substituiertem Heteroalkinyl, Cycloalkyl, Cycloalkenyl, substituiertem Cycloalkyl, substituiertem Cycloalkenyl, Halogen, Hydroxy, Alkoxy, Aryloxy, Carbonyl, Amino, Amido, Thio und Phosphino; bevorzugt ist B unabhängig voneinander ausgewählt aus C₁₋₆-Alkyl, C₁₋₆-Alkenyl, C₁₋₆-Alkinyl, C₃₋₈-Cycloalkyl, C₃₋₈-Cycloalkenyl, Alkoxy, Aryloxy, Amino und Amido.

Der Ausdruck unabhängig voneinander bedeutet, dass, wenn n 2 oder größer ist, die Substituenten B gleiche oder verschiedene Reste aus den genannten Gruppen sein können.

Unter Alkyl sind gemäß der vorliegenden Anmeldung verzweigte oder unverzweigte, gesättigte acyclische Kohlenwasserstoff-Reste zu verstehen. Bevorzugt werden Alkyl-Reste mit 1 bis 20 Kohlenstoffatomen, besonders bevorzugt mit 1 bis 6 Kohlenstoffatomen und insbesondere mit 1 bis 4 Kohlenstoffatomen eingesetzt. Beispiele für geeignete Alkyl-Reste sind Methyl, Ethyl, n-Propyl, i-Propyl, n-Butyl, t-Butyl und i-Butyl.

Unter Alkenyl sind gemäß der vorliegenden Anmeldung verzweigte oder unverzweigte acyclische Kohlenwasserstoff-Reste zu verstehen, die mindestens eine Kohlenstoff-Kohlenstoff-Doppelbindung aufweisen. Bevorzugt weisen die Alkenyl-Reste 2 bis 20 Kohlenstoffatome, besonders bevorzugt 2 bis 6 Kohlenstoffatome und insbesondere 2 bis 3 Kohlenstoffatome auf. Geeignete Alkenyl-Reste sind beispielsweise Vinyl und 2-Propenyl.

Unter Alkinyl sind gemäß der vorliegenden Anmeldung verzweigte oder unverzweigte acyclische Kohlenwasserstoff-Reste zu verstehen, die mindestens eine Kohlenstoff-Kohlenstoff-Dreifachbindung aufweisen. Vorzugsweise weisen die Alkinyl-Reste 2 bis 20 Kohlenstoffatome, besonders bevorzugt 1 bis 6 Kohlenstoffatome und insbesondere 2 bis 3 Kohlenstoffatome auf. Beispiele für geeignete Alkinyl-Reste sind Ethinyl und 2-Propinyl.

Unter substituiertem Alkyl, substituiertem Alkenyl und substituiertem Alkinyl sind Alkyl-Alkenyl- und Alkinyl-Reste zu verstehen, worin ein oder mehrere Wasserstoffatome, die an ein Kohlenstoffatom dieser Reste gebunden sind, durch eine andere Gruppe ersetzt sind. Beispiele für solche andere Gruppen sind Halogen, Aryl, substituiertes Aryl, Cycloalkyl, Cycloalkenyl, substituiertes Cycloalkyl, substituiertes Cycloalkenyl und Kombinationen davon. Beispiele für geeignete substituierte Alkyl-Reste sind Benzyl und Trifluormethyl.

Unter den Begriffen Heteroalkyl, Heteroalkenyl und Heteroalkinyl sind Alkyl- Alkenyl- und Alkinyl-Reste zu verstehen, worin ein oder mehrere der Kohlenstoffatome in der Kohlenstoffkette durch ein Heteroatom ausgewählt aus N, O und S ersetzt sind. Die Bindung zwischen dem Heteroatom und einem weiteren Kohlenstoffatom kann dabei gesättigt oder ungesättigt sein.

Unter Cycloalkyl sind gemäß der vorliegenden Anmeldung gesättigte cyclische nicht-aromatische Kohlenwasserstoff-Reste zu verstehen, die aus einem einzigen Ring oder mehreren kondensierten Ringen aufgebaut sind. Bevorzugt weisen die Cycloalkyl-Reste zwischen 3 und 8 Kohlenstoffatome und besonders bevorzugt zwischen 3 und 6 Kohlenstoffatome auf. Geeignete Cycloalkyl-Reste sind beispielsweise Cycloproyol, Cyclopentyl, Cyclohexyl, Cyclooctanyl und Bicyclooctyl.

Unter Cycloalkenyl sind gemäß der vorliegenden Anmeldung teilweise ungesättigte, cyclische nicht-aromatische Kohlenwasserstoff-Reste zu verstehen, die einen einzigen oder mehrere kondensierte Ringe aufweisen. Bevorzugt weisen die Cycloalkenyl-Reste 3 bis 8 Kohlenstoffatome und besonders bevorzugt 5 bis 6 Kohlenstoffatome auf. Geeignete Cycloalkenyl-Reste sind beispielsweise Cyclopentenyl, Cyclohexenyl und Cyclooctenyl.

Substituierte Cycloalkyl- und substituierte Cycloalkenyl-Reste sind Cycloalkyl- und Cycloalkenyl-Reste, worin ein oder mehrere Wasserstoffatome eines Kohlenstoffatoms des Kohlenstoffrings durch eine andere Gruppe ersetzt sind. Solche andere Gruppen sind beispielsweise Halogen, Alkyl, Alkenyl, Alkinyl, substituiertes Alkyl, substituiertes Alkenyl, substituiertes Alkinyl, Aryl, substituiertes Aryl, Cycloalkyl, Cycloalkenyl, substituiertes Cycloalkyl, substituiertes Cycloalkenyl, ein aliphatischer heterocyclischer Rest, ein substituierter aliphatischer heterocyclischer Rest, Heteroaryl, substituiertes Heteroaryl, Alkoxy, Aryloxy, Boryl, Phosphino, Amino, Silyl, Thio, Seleno und Kombinationen davon. Beispiele für substituierte Cycloalkyl und Cycloalkenyl-Reste sind 4-Dimethylaminocyclohexyl und 4,5-Dibromocyclohept-4-enyl.

Unter Aryl sind im Sinne der vorliegenden Anmeldung aromatische Reste zu verstehen, die einen einzelnen aromatischen Ring oder mehrere aromatische Ringe aufweisen, die kondensiert sind, über eine kovalente Bindung oder durch eine Verknüpfungseinheit, wie eine Methylen- oder Ethylen-Einheit verknüpft sind. Solche Verknüpfungseinheiten können auch Carbonyl-Einheiten, wie in Benzophenon, oder Sauerstoff-Einheiten, wie in Diphenylether, oder Stickstoff-Einheiten, wie in Diphenylamin, sein. Bevorzugt weisen die Aryl-Reste 6 bis 20 Kohlenstoffatome, besonders bevorzugt 6 bis 8 Kohlenstoffatome und insbesondere bevorzugt 6 Kohlenstoffatome auf. Beispiele für aromatische Ringe sind Phenyl, Naphthyl, Diphenyl, Diphenylether, Diphenylamin und Benzophenon.

Substituierte Aryl-Reste sind Aryl-Reste, worin ein oder mehrere Wasserstoffatome, die an Kohlenstoffatome des Aryl-Rests gebunden sind, durch eine oder mehrere Gruppen wie Alkyl, Alkenyl, Alkinyl, substituiertes Alkyl, substituiertes Alkenyl, substituiertes Alkinyl, Cycloalkyl, Cycloalkenyl, substituiertes Cycloalkyl, substituiertes Cycloalkenyl, Heterocyclo, substituiertes Hetereocyclo, Halogen, halogensubstituiertes Alkyl (z. B. CF₃), Hydroxy, Amino, Phosphino, Alkoxy und Thio ersetzt wird. Darüber hinaus können ein oder mehrere Wasserstoffatome, die an Kohlenstoffatome des Aryl-Rests gebunden sind, durch eine oder mehrere Gruppen wie gesättigte und/oder ungesättigte cyclische Kohlenwasserstoffe, die an den aromatischen Ring bzw. an die aromatischen Ringe kondensiert sein können oder durch eine Bindung verknüpft sein können, oder über eine geeignete Gruppe miteinander verknüpft sein können, ersetzt werden. Geeignete Gruppen sind die vorstehend beschriebenen.

Unter Heteroaryl sind gemäß der vorliegenden Anmeldung die vorstehend genannten Arylverbindungen zu verstehen, worin ein oder mehrere Kohlenstoffatome des Rests durch ein Heteroatom, z. B. N, O oder S ersetzt sind.

Unter Heterocyclo ist gemäß der vorliegenden Anmeldung ein gesättigter, teilweise ungesättigter oder ungesättigter cyclischer Rest zu verstehen, worin ein oder mehrere Kohlenstoffatome des Rests durch ein Heteroatom wie N, O oder S ersetzt sind. Beispiele für Heterocyclo-Reste sind Piperazinyl, Morpholinyl, Tetrahydropyranyl, Tetrahydrofuranyl, Piperidinyl, Pyrolidinyl, Oxazolinyl, Pyridyl, Pyrazyl, Pyridazyl, Pyrimidyl.

Substituierte Heterocyclo-Reste sind solche Heterocyclo-Reste, worin ein oder mehrere Wasserstoffatome, die an eines der Ringatome gebunden sind, durch eine oder mehrere Gruppen wie Halogen, Alkyl, substituiertes Alkyl, Aryl, substituiertes Aryl, Heteroaryl, substituiertes Heteroaryl, Alkoxy, Aryloxy, Boryl, Phosphino, Amino, Silyl, Thio, Seleno ersetzt sind.

Unter Alkoxy-Resten sind Reste der allgemeinen Formel -OZ¹ zu verstehen, worin Z¹ ausgewählt ist aus Alkyl, substituiertem Alkyl, Cycloalkyl, substituiertem Cycloalkyl, Heterocycloalkyl, substituiertem Heterocycloalkyl und Silyl. Geeignete Alkoxy-Reste sind beispielsweise Methoxy, Ethoxy, Benzyloxy und t-Butoxy.

Unter dem Begriff Aryloxy sind solche Reste der allgemeinen Formel -OZ² zu verstehen, worin Z² ausgewählt ist aus Aryl, substituiertem Aryl, Heteroaryl, substituiertem Heteroaryl und Kombinationen davon. Geeignete Aryloxy-Reste und Heteroaryloxy-Reste sind Phenoxy, substituiertes Phenoxy, 2-Pyridinoxy und 8-Chinolinoxy.

Unter Amino-Resten sind Reste der allgemeinen Formel -NZ³Z⁴ zu verstehen, worin Z³ und Z⁴ unabhängig voneinander ausgewählt sind aus Wasserstoff, Alkyl, substituiertem Alkyl, Cycloalkyl, substituiertem Cycloalkyl, Heterocycloalkyl, substituiertem Heterocycloalkyl, Aryl, substituiertem Aryl, Heteroaryl, substituiertem Heteroaryl, Alkoxy, Aryloxy und Silyl.

Bevorzugte in dem erfindungsgemäßen Aminierungsverfahren eingesetzte aromatische und heteroaromatische Kohlenwasserstoffe sind Benzol, Naphthalin, Diphenylmethane, Anthracen, Toluol, Xylol, Phenol und Anilin sowie Pyridin, Pyrazin, Pyridazin, Pyrimidin und Chinolin.

In einer bevorzugten Ausführungsform wird demnach mindestens ein Kohlenwasserstoff aus der Gruppe Benzol, Naphthalin, Diphenylmethane, Anthracen, Toluol, Xylol, Phenol und Anilin sowie Pyridin, Pyrazin, Pyridazin, Pyrimidin und Chinolin eingesetzt.

Es ist auch möglich, Mischungen der genannten aromatischen bzw. heteroaromatischen Kohlenwasserstoffe einzusetzen. Besonders bevorzugt wird mindestens ein aromatischer Kohlenwasserstoff aus der Gruppe Benzol, Naphthalin, Anthracen, Toluol, Xylol Phenol und Anilin, ganz besonders bevorzugt Benzol, Toluol, und Naphthalin eingesetzt.

Insbesondere bevorzugt wird Benzol in dem erfindungsgemäßen Verfahren eingesetzt.

Als aliphatischer Kohlenwasserstoff ist Methan für den Einsatz im erfindungsgemäßen Verfahren insbesondere bevorzugt.

### Aminierungsreagenz:

Erfindungsgemäß enthält der Eduktstrom E mindestens ein Aminierungsreagenz. Geeignete Aminierungsreagenzien sind solche, durch die mindestens eine Aminogruppe in den zur Direktaminierung eingesetzten Kohlenwasserstoff eingeführt wird. Beispiele für bevorzugte Aminierungsreagenzien sind Ammoniak, primäre und sekundäre Amine und Verbindungen, die unter den Reaktionsbedingungen Ammoniak abspalten. Es ist auch möglich Mischungen von zwei oder mehreren der vorstehend genannten Aminierungsreazien einzusetzen.

Insbesondere bevorzugtes Aminierungsreagenz ist Ammoniak.

### Aminokohlenwasserstoffe:

Im erfindungsgemäßen Verfahren wird ein Eduktstrom E, der mindestens einen Kohlenwasserstoff und mindestens ein Aminierungsreagenz enthält, zu einem Reaktionsgemisch R, das mindestens einen Aminokohlenwasserstoff und Wasserstoff enthält, umgesetzt. Dabei wird mindestens ein zum eingesetzten Kohlenwasserstoff korrespondierender Aminokohlenwasserstoff erhalten, der mindestens eine Aminogruppe mehr als der eingesetzte Kohlenwasserstoff enthält. Unter Aminokohlenwasserstoff ist im Rahmen der vorliegenden Erfindung demnach das Reaktionsprodukt der im Verfahren eingesetzten Kohlenwasserstoffe mit dem Aminierungsreagenz zu verstehen. Hierbei wird mindestens eine Aminogruppe von dem Aminierungsreagenz auf den Kohlenwasserstoff übertragen. In einer bevorzugten Ausführungsform werden 1 bis 6 Aminogruppen, in einer besonders bevorzugten Ausführungsform 1 bis 3 Aminogruppen, ganz besonders bevorzugt 1 bis 2 Aminogruppen und insbesondere bevorzugt 1 Aminogruppe auf den Kohlenwasserstoff übertragen. Die Anzahl der übertragenen Aminogruppen lässt sich durch das molare Verhältnis zwischen Aminierungsreagenz und zu aminierendem Kohlenwasserstoff sowie durch die Reaktionstemperatur steuern.

Typischerweise beträgt das Verhältnis von Aminierungsreagenz zu Kohlenwasserstoff 0,5 bis 9, bevorzugt 1 bis 5, besonders bevorzugt 1,5 bis 3.

Für den Fall, dass in das erfindungsgemäße Verfahren als Kohlenwasserstoff Benzol und als Aminierungsreagenz Ammoniak in einem Molverhältnis im Bereich von 1 bis 9 eingesetzt werden, wird als Aminokohlenwasserstoff Anilin erhalten.

Für den Fall, dass in das erfindungsgemäße Verfahren als Kohlenwasserstoff Toluol und als Aminierungsreagenz Ammoniak in einem Molverhältnis im Bereich von 1 bis 9 eingesetzt wird, wird als Aminokohlewasserstoff Toluoldiamin erhalten.

Für den Fall, dass in das erfindungsgemäße Verfahren als Kohlenwasserstoff Methan und als Aminierungsreagenz Ammoniak in einem Molverhältnis im Bereich von 1 bis 9 eingesetzt wird, wird als Aminokohlewasserstoff Methylamin, Dimethylamin oder Trimethylamin oder ein Gemisch aus zwei oder mehreren der vorstehend genannten Amine erhalten.

In einer besonderen Ausführungsform wird Benzol mit Ammoniak zu Anilin umgesetzt. In einer weitern besondern Ausführungsform wird Toluol mit Ammoniak zu Toluoldiamin umgesetzt.

### Katalysatoren:

Die Direktaminierung wird in Gegenwart mindestens eines Katalysators durchgeführt.

Als Katalysatoren sind prinzipiell alle bekannten Aminierungskatalysatoren geeignet. Als Katalysatoren können die für die direkte Aminierung von Kohlenwasserstoffen, insbesondere die für die direkte Aminierung von Benzol mit Ammoniak zu Anilin bekannten Katalysatoren eingesetzt werden. Derartige Katalysatoren sind in der Patentliteratur beispielsweise in WO 2007/099028, WO 2007/096297, WO 2000/69804, WO 2008/009668, WO 2007/025882, US 3,919,155, US 3,929,889, US 4,001,260, US 4,031,106, und WO 99/10311 beschrieben. Da gemäß dem erfindungsgemäßen Verfahren die Entfernung des Wasserstoffs elektrochemisch und nicht durch chemische Umwandlung im Reaktionssystem erfolgt, können auch Katalysatoren eingesetzt werden, die keine gegenüber Wasserstoff reaktive Komponenten aufweisen.

Als Katalysatoren können beispielsweise übliche Metallkatalysatoren auf der Basis von Nickel, Eisen, Kobalt, Kupfer, Edelmetallen oder Legierungen dieser genannten Metalle eingesetzt werden. Bevorzugte Edelmetalle (EM) sind Ru, Rh, Pd, Ag, Ir, Pt und Au. In einer besonderen Ausführungsform werden die Edelmetalle Ru und Rh nicht alleine, sondern in Legierung mit einem oder mehreren anderen Übergangsmetallen, wie Co, Cu, Fe und Nickel eingesetzt. Beispiele für geeignete Katalysatoren sind geträgerte NiCuEM; CoCuEM; NiCoCuEM, NiMoEM, NiCrEM, NiReEM, CoMoEM, CoCrEM, CoReEM, FeCuEM, FeCoCuEM, FeMoEM, FeReEM Legierungen. Dabei ist EM ein Edelmetall, bevorzugt Pt, Pd, Ag, Ir, insbesondere bevorzugt Ag und/oder Ir. Weiter insbesondere bevorzugt ist NiCuEM, wobei EM aus Pt, Pd, Ag und/oder Ir ausgewählt ist.

In einer Ausführungsform dient der auf der Retentatseite eingesetzte Elektrokatalysator (Elektrode) gleichzeitig als Katalysator für die Umsetzung von Kohlenwasserstoff zu Aminokohlenwasserstoff (Aminierungskatalysator). In einer weiteren Ausführungsform kann der Aminierungskatalysator direkt auf dem Elektrokatalysator angebracht werden. In diesem Fall wird der bei der Reaktion freigesetzte Wasserstoff direkt von der Katalysatoroberfläche des Aminierungskatalysators durch die protonenleitende Membran abgeführt.

Der Katalysator kann in allgemein üblicher Form, beispielsweise als Pulver oder für den Einsatz im Festbett in Form von Strängen, Kugeln, Tabletten oder Ringe eingesetzt werden. Die katalytisch aktiven Bestandteile können auf einem Trägermaterial vorliegen. Als Trägermaterial kommen beispielsweise anorganische Oxide, beispielsweise ZrO₂, SiO₂, Al₂O₃, MgO, TiO₂, B₂O₃, CaO, ZnO, BaO, ThO₂, CeO₂, Y₂O₃ und Mischungen dieser Oxide, wie Magnesium-Aluminium-oxid, bevorzugt TiO₂, ZrO₂, Al₂O₃, Magnesium-Aluminium-oxid und SiO₂ in Betracht. Besonders bevorzugt sind Al₂O₃, ZrO₂ und Magnesium-Aluminium-oxid. Unter ZrO₂ wird sowohl reines ZrO₂ als auch das normale Hf-enthaltende ZrO₂ verstanden.

Die in dem erfindungsgemäßen Verfahren bevorzugt eingesetzten Katalysatoren können regeneriert werden, z.B. indem man eine reduktive Atmosphäre über den Katalysator leitet oder zuerst eine oxidative und anschließend eine reduktive Atmosphäre über oder durch das Katalysatorbett führt. Als reduktive Atmosphäre wird vorzugsweise eine H₂-Atmosphäre verwendet.

### Reaktionsbedingungen Direktaminierung:

Die Direktaminierung kann unter oxidativen oder nicht oxidativen Bedingungen durchgeführt werden. Die Direktaminierung kann darüber hinaus unter katalytischen oder nicht katalytischen Bedingungen durchgeführt werden.

In einer bevorzugten Ausführungsform findet die Direktaminierung in Gegenwart eines Katalysators unter nicht-oxidativen Bedingungen statt.

Nicht-oxidativ gemäß der vorliegenden Erfindung bedeutet in Bezug auf die Direktaminierung, dass die Konzentration von Oxidationsmitteln wie Sauerstoff oder Stickoxiden in den eingesetzten Edukten (Eduktstrom E) unterhalb von 5 Gew.-%, bevorzugt unterhalb von 1 Gew.-%, besonders bevorzugt unterhalb von 0,1 Gew.-% liegt (jeweils bezogen auf das Gesamtgewicht des Eduktstroms E). Unter Eduktstrom E wird im Rahmen der vorliegenden Erfindung der Strom verstanden, der zum Reaktor geführt wird und mindestens einen Kohlenwasserstoff und mindestens ein Aminierungsreagenz enthält. Ganz besonders bevorzugt ist der Eduktstrom E frei von Sauerstoff. Ebenfalls besonders bevorzugt ist eine Konzentration an Oxidationsmittel im Eduktstrom E, die gleichgroß oder geringer ist als die Konzentration an Oxidationsmitteln in der Quelle, aus der die eingesetzten Kohlenwasserstoffe und Aminierungsreagenzien stammen.

Die Reaktionsbedingungen in den erfindungsgemäßen Direktaminierungsverfahren sind unter anderem von dem zu aminierenden Kohlenwasserstoff und dem eingesetzten Katalysator abhängig.

Die Direktaminierung erfolgt im Allgemeinen bei Temperaturen von 20 bis 800 °C, bevorzugt 50 bis 700 °C, besonders bevorzugt 70 bis 350 °C.

Der Reaktionsdruck beträgt bei der Direktaminierung vorzugsweise 0,5 bis 40 bar, bevorzugt 1 bis 6 bar, besonders bevorzugt 1 bis 3 bar, insbesondere Atmosphärendruck.

Die Verweilzeit bei diskontinuierlicher Verfahrensführung beträgt in dem erfindungsgemäßen Aminierungsverfahren im Allgemeinen 15 Minuten bis 8 Stunden, bevorzugt 15 Minuten bis 4 Stunden, besonders bevorzugt 15 Minuten bis 1 Stunde. Bei Durchführung in einem kontinuierlichen Verfahren beträgt die Verweilzeit im Allgemeinen 0,1 Sekunden bis 20 Minuten, bevorzugt 0,5 Sekunden bis 10 Minuten. Für die bevorzugten kontinuierlichen Verfahren bedeutet "Verweilzeit" in diesem Zusammenhang die Verweilzeit des Eduktstroms E am Katalysator, für Festbettkatalysatoren somit die Verweilzeit im Katalysatorbett, für Wirbelschichtreaktoren wird der Syntheseteil des Reaktors (Teil des Reaktors, wo der Katalysator lokalisiert ist) betrachtet.

Die relative Menge des eingesetzten Kohlenwasserstoffs und des Aminierungsreagenz ist abhängig von der durchgeführten Aminierungsreaktion und den Reaktionsbedingungen. Im Allgemeinen werden mindestens stöchiometrische Mengen des Kohlenwasserstoffs und des Aminnierungsreagenz eingesetzt. Bevorzugt wird einer der Reaktionspartner im stöchiometrischen Überschuss eingesetzen, um eine Gleichgewichtsverschiebung auf die Seite des gewünschten Produkts und somit einen höheren Umsatz zu erreichen. Bevorzugt wird das Aminnierungsreagenz im stöchiometrischen Überschuss im Bezug auf den Kohlenwasserstoff eingesetzt. Das Verhältnis Aminierungsreagenz zu Kohlenwasserstoff beträgt 0,5 bis 9, bevorzugt 1 bis 5, besonders bevorzugt 1,5 bis 3.

### Wasserstoffabtrennung:

Im erfindungsgemäßen Verfahren wird mindestens ein Teil des im Reaktionsgemisch R enthaltenen Wasserstoffs elektrochemisch abgetrennt und unter Erzeugung von elektrischem Strom mit Sauerstoff zu Wasser umgesetzt. Unter dem Reaktionsgemisch R ist das Gemisch zu verstehen, das durch die chemische Umsetzung von mindestens einem Kohlenwasserstoff mit mindestens einem Aminierungsreagenz entsteht. Das Reaktionsgemisch enthält daher üblicherweise als Reaktionsprodukte den oder die korrespondierenden Aminokohlenwasserstoffe und Wasserstoff. Gegebenenfalls kann das Reaktionsgemisch R darüber hinaus nicht umgesetzte Edukte enthalten. Der Wasserstoff wird mittels einer gasdichten Membran-Elektroden-Assembly abgetrennt, wobei der abzutrennende Wasserstoff in Form von Protonen durch die Membran transportiert wird. Die Elektroden mit der dazwischen angeordneten Membran werden Membran-Elektroden-Assembly (MEA) genannt. Das Reaktionsgemisch R wird auf einer Seite der Membran entlang geführt. Diese Seite wird im Folgenden Retentatseite genannt. Auf der anderen Seite der Membran, im Folgenden als Permeatseite bezeichnet, wird ein Sauerstoff enthaltender Strom O entlanggeführt. Erfindungsgemäß weist die MEA mindestens eine selektiv protonenleitende Membran auf. Die Membran weist auf jeder Seite mindestens einen Elektrodenkatalysator auf, wobei im Rahmen dieser Beschreibung der auf der Retentatseite befindliche Elektrodenkatalysator als Anodenkatalysator, der auf der Permeatseite befindliche Elektrodenkatalysator als Kathodenkatalysator bezeichnet wird. Auf der Retentatseite wird der Wasserstoff am Anodenkatalysator zu Protonen oxidiert, diese durchqueren die Membran und reagieren auf der Permeatseite am Kathodenkatalysator mit dem Sauerstoff zu Wasser. Die treibende Kraft ist die Reduktion des Sauerstoffs. Bei der Gesamtreaktion wird Energie in Form von Wärme und durch Zwischenschalten eines Verbrauchers in Form von elektrischem Strom frei.

In einer bevorzugten Ausführungsform wird der Wasserstoff direkt aus dem Reaktionsgemisch R abgetrennt. Weiterhin bevorzugt wird der Wasserstoff direkt aus der Reaktionszone RZ abgetrennt, in der das Reaktionsgemisch R gebildet wird.

Unter Reaktionszone RZ im Rahmen der vorliegenden Erfindung ist der Bereich zu verstehen, in dem die chemische Umsetzung von mindestens einem Kohlenwasserstoff und mindestens einem Aminierungsreagenz zum Reaktionsgemisch R stattfindet.

Die Abtrennung des Wasserstoffs nach dem erfindungsgemäßen Verfahrens kann bei Temperaturen von 20 bis 800 °C, bevorzugt von 50 bis 700 °C, besonders bevorzugt von 70 bis 350 °C durchgeführt werden. Bei Verwendung von MEAs basierend auf Polybenzimidazol und Phosphorsäure wird die Abtrennung bevorzugt bei 130 bis 200 °C vorgenommen. Bei der Verwendung von keramischen Membranen, z.B. auf Basis von Ammoniumpolyphosphat, kann bei Temperaturen von 250 bis 350 °C gearbeitet werden.

Die Abtrennung des Wasserstoffs nach dem erfindungsgemäßen Verfahrens wird vorzugsweise bei Drücken von 0,5 bis 40 bar, bevorzugt von 1 bis 6 bar, besonders bevorzugt von 1 bis 3 bar, insbesondere bei Atmosphärendruck vorgenommen. Gemäß einer bevorzugten Ausführungsform der Erfindung liegt der Druckunterschied zwischen der Retentat- und der Permeatseite der Membran unter 1 bar, bevorzugt unter 0,5 bar, besonders bevorzugt besteht kein Druckunterschied.

Der Sauerstoff enthaltende Strom O enthält erfindungsgemäß mindestens 15 mol-%, bevorzugt mindestens 20 mol-% Sauerstoff. In einer bevorzugten Ausführungsform wird Luft als Sauerstoff enthaltender Strom O eingesetzt oder mit Sauerstoff angereichte Luft. Die Luft wird üblicherweise ungereinigt verwendet.

Erfindungsgemäß wird mindestens ein Teil des bei der Direktaminierung entstandenen Wasserstoffs abgetrennt. Bevorzugt werden mindestens 30 %, besonders bevorzugt mindestens 50 %, besonders bevorzugt mindestens 70 % und ganz besonders bevorzugt mindestens 95 %, insbesondere mindestens 98 % abgetrennt.

Gemäß einer bevorzugten Ausführungsform wird die Abtrennung des Wasserstoffs aus dem Reaktionsgemisch R in einem Reaktor durchgeführt, der mit mindestens einer gasdichten MEA in der Art ausgestattet ist, dass die Reaktionszone RZ auf der Retentatseite der Membran liegt bzw. diese bildet. Die Abtrennung kann in einer Ausführungsform beispielsweise in einem Reaktor, dessen Außenwände zumindest teilweise aus gasdichten MEAs gebildet sind, durchgeführt werden.

### Reaktoren:

Im erfindungsgemäßen Verfahren können alle Reaktortypen eingesetzt werden, die für Direktaminierungsreaktionen geeignet sind, und die durch mindestens eine gasdichte MEA modifiziert wurden.

Geeignete Reaktoren sind somit Rohr-Reaktoren, Festbettreaktoren, Membranereaktoren, Wirbelschichtreaktoren, Wanderbetten, zirkulierende Wirbelschichten, Salzbadreaktoren, Plattenwärmetauscher-Reaktoren, Hordenreaktoren mit mehreren Horden mit/oder ohne Wärmetausch bzw. Abzug/Zufuhr von Teilströmen zwischen den Horden, in möglichen Ausführungen als Radialstrom- oder Axialstromreaktoren, wobei jeweils der für die gewünschten Reaktionsbedingungen (wie Temperatur, Druck und Verweilzeit) geeignete Reaktor eingesetzt wird. Die Reaktoren können jeweils als einzelner Reaktor (single reactor), als Serie von einzelnen Reaktoren und/oder in Form von zwei oder mehr parallelen Reaktoren eingesetzt werden. Das erfindungsgemäße Verfahren kann als semi-kontinuierliche Reaktion oder kontinuierliche Reaktion durchgeführt werden. Der spezielle Reaktoraufbau und die Durchführung der Reaktion können in Abhängigkeit von dem durchzuführenden Aminierungsverfahren, dem Aggregatzustand des zu aminierenden aromatischen Kohlenwasserstoffs, den erforderlichen Reaktionszeiten und der Natur des eingesetzten Katalysators variieren. Bevorzugt wird das erfindungsgemäße Verfahren zur Direktaminierung in einem Festbettreaktor oder Wirbelschichtreaktor durchgeführt, die erfindungsgemäß mit mindestens einer MEA modifiziert wurden.

### Elektrodenkatalysatoren:

Die Funktion von Elektrodenkatalysators wird z. B. in Journal of Power Sources 177 (2008), 478-484, K.A. Perry, G.A. Eisman, B.C. Benicewicz "Electrochemical hydrogen pumping using a high-temperature polybenzimidazole (PBI) membrane" beschrieben.

Um einen guten Kontakt der Membran mit dem auf der Retentatseite befindlichen Wasserstoff und einen guten Abtransport des abgetrennten Wasserstoffs auf der Permeatseite zu gewährleisten, sind die Elektrodenschichten üblicherweise mit Gasverteilerschichten kontaktiert. Diese sind zum Beispiel Platten mit einer gitterartigen Oberflächenstruktur aus einem System feiner Kanäle oder Schichten aus porösem Material wie Vlies, Gewebe oder Papier. Die Gesamtheit von Gasverteilerschicht und Elektrodenschicht wird im Allgemeinen als Gasdiffusionselektrode (GDE) bezeichnet. Durch die Gasverteilerschicht wird der abzutrennende Wasserstoff auf der Retentatseite nahe an die Membran und den Anodenkatalysator geführt und auf der Permeatseite der Abtransport des gebildeten Wasserstoffs erleichtert.

Je nach Ausführungsform der Erfindung kann die Anode gleichzeitig auch als Anodenkatalysator und die Kathode gleichzeitig auch als Kathodenkatalysator dienen. Es können jedoch auch jeweils unterschiedliche Materialien für die Anode und den Anodenkatalysator bzw. die Kathode und den Kathodenkatalysator verwendet werden.

In einer Ausführungsform der Erfindung kann der Anodenkatalysator gleichzeitig auch als Aminierungskatalysator dienen. In diesem Fall wird der Anodenkatalysator aus mindestens einem Material der vorstehend genannten Aminierungskatalysatoren ausgebildet.

Zur Herstellung der Elektroden können die üblichen, dem Fachmann bekannten Materialien verwendet werden, beispielsweise Pt, Pd, Cu, Ni, Ru, Co, Cr, Fe, Mn, V, W, Wolframcarbid, Mo, Molybdäncarbid, Zr, Rh, Ag, Ir, Au, Re, Y, Nb, elektrisch leitende Formen von Kohlenstoff wie Ruß, Graphit und Nanoröhrchen sowie Legierungen und Mischungen der vorstehend genannten Elemente.

Die Anode und die Kathode können aus dem gleichen Material oder aus unterschiedlichen Materialien hergestellt sein. Der Anodenkatalysator und der Kathodenkatalysator können aus den gleichen oder aus unterschiedlichen Materialien ausgewählt sein. Besonders bevorzugt sind als Anoden/Kathoden-Kombination Pt/Pt, Pd/Pd, Pt/Pd, Pd/Pt, Pd/Cu, Pd/Ag, Ni/Pt, Ni/Ni und Fe/Fe.

Als Elektrodenkatalysatormaterial können die üblichen, dem Fachmann bekannten Verbindungen und Elemente verwendet werden, die die Dissoziation von molekularem Wasserstoff in atomaren Wasserstoff, die Oxidation von Wasserstoff zu Protonen sowie die Reduktion von Protonen zu Wasserstoff katalysieren können. Geeignet sind beispielsweise Pd, Pt, Cu, Ni, Ru, Fe, Co, Cr, Mn, V, W, Wolframcarbid, Mo, Molybdäncarbid, Zr, Rh, Ag, Ir, Au, Re, Y, Nb sowie Legierungen und Mischungen davon, bevorzugt sind erfindungsgemäß Pd, Pt und Ni. Die vorstehend als Elektrodenkatalysatormaterial genannten Elemente und Verbindungen können auch geträgert vorliegen, bevorzugt wird dabei Kohlenstoff als Träger eingesetzt.

Gemäß einer bevorzugten Ausführungsform der vorliegenden Erfindung werden bevorzugt Kohlenstoff als leitendes Material enthaltende Elektroden verwendet. Dabei wird der Kohlenstoff und ein Elektrodenkatalysator bevorzugt auf einem porösen Träger wie Vlies, Gewebe oder Papier aufgebracht. Der Kohlenstoff kann dabei mit dem Katalysator vermischt oder zunächst der Kohlenstoff und anschließend der Katalysator aufgebracht werden.

Gemäß einer weiteren Ausführungsform der Erfindung wird das als Elektrode eingesetzte elektrisch leitende Material und der Elektrodenkatalysator direkt auf der Membran aufgebracht.

### Membranen:

Die Membran ist vorzugsweise als Platte oder als Rohr ausgebildet, wobei die üblichen, aus dem Stand der Technik zur Auftrennung von Gasgemischen bekannten Membrananordnungen verwendet werden können beispielsweise Rohrbündel- oder Steckplattenmembran.

Die erfindungsgemäß eingesetzte MEA ist gasdicht, das heißt sie weist praktisch keine Porosität auf, durch die Gase in atomarer oder molekularer Form von einer Seite auf die andere Seite der MEA gelangen können, noch weist sie Mechanismen auf, durch die Gase unselektiv beispielsweise durch Adsorption, Lösung in der Membran, Diffusion und Desorption durch die MEA transportiert werden können. Die Dichtigkeit der Membran-Elektroden-Assembly (MEA) kann entweder durch eine gasdichte Membran, durch eine gasdichte Elektrode, oder einen gasdichten Elektrodenkatalysator sowie durch eine Kombination davon gewährleistet werden. So kann als gasdichte Elektrode zum Beispiel eine dünne metallische Folie verwendet werden, beispielsweise eine Pd-, Pd-Ag- oder Pd-Cu-Folie. Weiterhin leitet die erfindungsgemäß eingesetzte Membran selektiv Protonen, das heißt insbesondere, dass sie nicht elektronenleitend ist.

Erfindungsgemäß können für die Membranen alle dem Fachmann bekannten Materialien eingesetzt werden, aus denen sich selektiv protonenleitende Membranen formen lassen. Hierzu gehören beispielsweise die von J. W. Phair und S. P. S. Badwal in Ionics (2006) 12, Seiten 103 - 115 aufgeführten Materialien. Auch selektiv protonenleitende Membranen, wie sie aus der Brennstoffzellentechnik bekannt sind, können erfindungsgemäß verwendet werden.

Beispielsweise können die nachfolgenden keramischen Membranen wie bestimmte Heteropolysäuren wie H₃Sb₃B₂O₁₄ • 10H₂O, H₂Ti₄O₉ • 12H₂O und HSbP₂O₈ • 10 H₂O; saure Zirkoniumsilikate, -phosphate und -phosphonate in Schichtstruktur wie K₂ZrSi₃O₉, K₂ZrSi₃O₉, alpha-Zr(HPO₄)₂ • nH₂O, gamma-Zr(PO₄)-(H₂PO₄) • 2H₂O, alpha- und gamma-Zr-Sulfophenylphosphonat oder Sulfoarylphosphonat; nicht geschichtete Oxidhydrate wie Antimonsäure (Sb₂O₅ • 2H₂O), V₂O₅ • nH₂O, ZrO₂ • nH₂O, SnO₂ • nH₂O und Ce(HPO₄)₂ • nH₂O eingesetzt werden. Weiterhin können Oxosäuren und Salze, die zum Beispiel Sulfat-, Selenat-, Phosphat-, Arsenat-, Nitrat-Gruppen usw. enthalten, verwendet werden. Besonders geeignet sind Oxoanionensysteme auf Basis von Phosphaten oder komplexen Heteropolysäuren wie Polyphosphatgläser, Aluminiumpolyphosphat, Ammoniumpolyphosphat und Polyphosphatzusammensetzungen wie NH₄PO₃/(NH₄)₂SiP₄O₁₃ und NH₄PO₃/TiP₂O₇. Weiterhin sind oxidische Materialien einsetzbar, wie Brownmillerite, Fluorite und Phosphate mit Apatitstruktur, Pyrochlor-Mineralien und Perovskite. Generell können alle protonenleitenden Materialien, wie z.B. auch Zeolithe, Alumosilikate, xAl₂O₃(1-x)SiO₂, SnP₂O₇, Sn₁₋ₓInₓP₂O₇ (X = 0.0 - 0.2), eingesetzt werden.

Perovskite weisen die Grundformel AB₁₋ₓMₓO_{3-y} auf, wobei M ein trivalentes Seltenerdenelement ist, das zur Dotierung dient, und y den Sauerstoffmangel im Perovskitoxid-Gitter bezeichnet. A kann beispielsweise ausgewählt werden aus Mg, Ca, Sr und Ba. B kann unter anderem ausgewählt werden aus CeZr und Ti. Für A, B und M können auch unabhängig voneinander verschiedene Elemente aus den jeweiligen Gruppen gewählt werden.

Weiterhin können strukturell modifizierte Gläser eingesetzt werden wie Chalcogenid-Gläser, PbO-SiO₂, BaO-SiO₂ und CaO-SiO₂.

Weitere geeignete protonenleitende Keramiken und Oxide sind beispielsweise in Solid State lonics 125, (1999), 271-278; Journal of Power Sources 180, (2008), 15-22; lonics 12, (2006), 103-115; Journal of Power Sources 179 (2008) 92-95; Journal of Power Sources 176 (2008) 122-127 und Electrochemistry Communications 10 (2008) 1005-1007 beschrieben.

Beispiele für protonenleitende Keramiken und Oxide sind SrCeO₃, BaCeO₃, Yb:SrCeO₃, Nd:BaCeO₃, Gd:BaCeO₃, Sm:BaCe0₃, BaCaNd0₉, Y:BaCeO₃, Y:BaZrCeO₃, Pr-dotiertes Y:BaCeO₃, Gd:BaCe0₃, BaCe_{0,9}Y_{0,1}O_{2,95} (BYC), SrCe_{0,95}Yb_{0,05}O_{3-α}, BaCe_{0,9}Nd_{0,10}O_{3-α}, CaZr_{0,96}In_{0,04}O_{3-α}, (α bezeichnet die Anzahl der Sauerstofffehlstellen pro Formeleinheit des Oxides vom Perowskittyp); Sr-dotiertes La₃P₃O₉, Sr-dotiertes LaPO₄, BaCe_{0,9}Y_{0,1}O_{3-α} (BCY), BaZr_{0,9}Y_{0,1}O_{3-α} (BZY), Ba₃Ca_{1,18}Nb_{1,82}O_{8,73} (BCN18), (La_{1,95}Ca_{0,05})Zr₂O_{7-α}, La₂Ce₂O₇, Eu₂Zr₂O₇, H₂S/(B₂S₃ oder Ga₂S₃)/GeS₂, SiS₂, As₂S₃ oder Csl; BaCe_{0,8}Gd_{0,2}O_{3-α} (BCGO); Gd-dotierte BaCeO₃ wie BaCe_{0,85}Y_{0,15}O_{3-α}, (BCY15) und BaCe_{0,8}Sm_{0,2}O_{3-α}, xAl₂O₃ (1-X)SiO₂, SnP₂O₇, Sn₁₋ₓInₓP₂O₇ (x = 0.0 - 0.2).

Eine weitere Klasse Materialien, die sich für die Herstellung von gasdichten und selektiv protonenleitenden Membranen eignen, sind Polymermembranen. Geeignete Polymere sind sulfonierte Polyetheretherketone (S-PEEK), sulfonierte Polybenzoimidazole (S-PBI) und sulfonierte Fluorkohlenwasserstoffpolymere (NAFION^{®}). Weiterhin können perflurierte Polysulfonsäuren, Polymere auf Styrolbasis, Poly(arylenether), Polyimide und Polyphosphazene eingesetzt werden. Es können auch Polybenzamidazolen eingesetzt werden, die auf Polybenzimidazol und Phosphorsäure basieren, wie sie beispielsweise unter dem Namen Celtec-P^{®} von der BASF SE vertrieben werden.

Erfindungsgemäß werden als Materialien für die selektiv protonenleitende Membran bevorzugt die vorstehend genannten keramischen Membranen eingesetzt.

Bei Verwendung von Polymermembranen werden diese üblicherweise durch die Gegenwart von etwa 0,5 bis 30 Vol.-% Wasser auf mindestens einer Seite der Membran befeuchtet.

### Aufarbeitung des Produktstroms:

Nach der Abtrennung des Wasserstoffs aus dem Reaktionsgemisch R mittels mindestens einer MEA wird ein Produktstrom P erhalten. Dieser enthält mindestens einen Aminokohlenwasserstoff und gegebenenfalls nicht umgesetzte Edukte, wie Kohlenwasserstoffe und Aminierungsreagenzien, sowie gegebenenfalls nicht abgetrennten Wasserstoff. In einer bevorzugten Ausführungsform enthält der Produktstrom P weniger als 500, bevorzugt weniger als 200 und insbesondere bevorzugt weniger als 100 ppm Wasserstoff.

Gegebenenfalls kann weiterer Wasserstoff aus dem Produktstrom P abgetrennt werden. Dazu wird der Produktstrom P in einem nachgelagerten Schritt erneut mit einem oder mehreren MEAs in Kontakt gebracht. In einer bevorzugten Ausführungsform erfolgt die Abtrennung des Wasserstoffs aus dem Reaktionsgemisch jedoch so vollständig, dass eine nachgelagerte Abtrennung von Wasserstoff aus dem Produktstrom P nicht notwendig ist.

In einer Verfahrensvariante (Variante I) wird der Aminokohlenwasserstoff und das Aminierungsreagenz aus dem Produktstrom P abgetrennt. Die Reihenfolge der Abtrennung ist dabei frei wählbar. Bevorzugt wird jedoch erst das Aminierungsreagenz und dann der Aminokohlenwasserstoff abgetrennt. Der so erhaltene aufgearbeitete Strom S1 enthält den nicht umgesetzten Kohlenwasserstoff. In einer bevorzugten Ausführungsform wird dieser erneut in die Direktaminierung eingesetzt. Der Kohlenwasserstoff aus Strom S1 wird dazu entweder dem Eduktstrom E zugemischt oder direkt der Reaktionszone zurückgeführt. Die Abtrennung von Aminokohlenwasserstoff und Aminierungsreagenz kann nach allgemein bekannten, dem Fachmann geläufigen Methoden erfolgen, beispielsweise durch Kondensation, Destillation oder Extraktion. Die Wahl des Druck- und Temperaturbereichs richtet sich nach den physikalischen Eigenschaften der aufzutrennenden Verbindungen und ist dem Fachmann bekannt.

So kann der Produktstrom P auf 50 °C bis 250 °C, vorzugsweise auf 70 °C bis 200 °C, besonders bevorzugt auf 80 °C bis 150 °C, bei Drücken im Bereich von 0 bis 5 bar, bevorzugt 0,5 bis 2 bar, besonders bevorzugt 0,8 bis 1,5 bar und insbesondere bei Normaldruck abgekühlt werden. Hierbei kondensieren die Aminokohlenwasserstoffe, während nicht umgesetzter Kohlenwasserstoff und Aminierungsreagenz und gegebenenfalls noch vorhandener Wasserstoff gasförmig vorliegen und somit nach üblichen Methoden beispielsweise mit einem Gas-Flüssig-Abscheider abgetrennt werden können.

Die so erhaltenen flüssigen Bestandteile enthalten den Aminokohlenwasserstoff und nicht umgesetzten Kohlenwasserstoff, die Auftrennung von Aminokohlenwasserstoff und Kohlenwasserstoff erfolgt ebenfalls nach dem Fachmann bekannten Methoden, wie Destillation, Rektifikation oder Säureextraktion. Die zum rückgeführten aufgearbeiteten Strom S1 zugefahrenen Mengen an Kohlenwasserstoff und Aminierungsreagenz werden so gewählt, dass die für die Direktaminierung erforderlichen Molverhältnisse von Kohlenwasserstoff und Aminierungsreagenz eingehalten werden.

Für den Fall der Direktaminierung von Benzol und Ammoniak zu Anilin und Wasserstoff enthält der Produktstrom P im Wesentlichen Anilin, nicht umgesetztes Benzol und Ammoniak und gegebenenfalls Nebenprodukte und Reste von Wasserstoff. Nach Variante I wird der Produktstrom P zunächst durch Kondensation in eine gasförmige Phase, enthaltend Ammoniak und gegebenenfalls Reste von Wasserstoff, und eine flüssige Phase, enthaltend Anilin und Benzol, aufgetrennt. Die flüssige Phase wird nachfolgend durch Destillation, Rektifikation oder Säureextraktion in Anilin und Benzol aufgetrennt. Das Benzol (Strom S1) wird erneut in die Direktaminierung eingesetzt. Das so erhaltene Anilin kann gegebenenfalls weiteren Aufarbeitungsschritten unterzogen werden.

Gemäß einer weiteren Verfahrensvariante (Variante II) wird der Produktstrom P erneut in die Direktaminierung eingesetzt. Der Produktstrom P kann hierzu dem Edutkstrom E zugemischt werden oder separat direkt in die Reaktionszone RZ eingeleitet werden. In einer bevorzugten Ausführungsform wird der Produktstrom P solange zurück in die Reaktionszone RZ geführt, bis sich im Produktstrom P eine Konzentration an Aminokohlenwasserstoff angereichert hat, die eine wirtschaftliche Aufarbeitung ermöglicht. Bevorzugt wird der Produktstrom P dazu ein- bis zwanzigmal, bevorzugt ein- bis zehnmal, besonders bevorzugt ein- bis fünfmal, und insbesondere bevorzugt ein- bis dreimal zur Reaktionszone RZ zurückgeführt. Bei dieser Fahrweise werden die zum rückgeführten Produktstrom P zugefahrenen Mengen an Kohlenwasserstoff und Aminierungsreagenz so gewählt, dass die für die Direktaminierung erforderlichen Molverhältnisse von Kohlenwasserstoff und Aminierungsreagenz eingehalten werden.

Die Aufarbeitung und Abtrennung des Aminokohlenwasserstoffs erfolgt gemäß dem unter Variante I beschriebenen Verfahren.

Gemäß einer weiteren Verfahrensvariante (Variante III) wird aus dem Produktstrom P der Aminokohlenwasserstoff abgetrennt. Der so erhaltene aufgearbeitete Strom S2 enthält in dieser Verfahrensvariante nicht umgesetzten Kohlenwasserstoff und Aminierungsreagenz. Dieser aufgearbeitete Strom S2 kann erneut in die Direktaminierung eingesetzt werden. Der aufgearbeitete Produktstrom kann hierzu dem Eduktstrom E zugemischt werden oder direkt in die Reaktionszone RZ eingeleitet werden. Die Abtrennung des Aminokohlenwasserstoffs erfolgt nach dem Fachmann bekannten Methoden, beispielsweise durch Kondensation, Destillation oder Säureextraktion. Bevorzugt wird in Variante III der Aminokohlenwasserstoff durch Säureextraktion aus dem Produktstrom P entfernt. Die zum rückgeführten Produktstrom P zugefahrenen Mengen an Kohlenwasserstoff und Aminierungsreagenz werden so gewählt, dass die für die Direktaminierung erforderlichen Molverhältnisse von Kohlenwasserstoff und Aminierungsreagenz eingehalten werden.

Die Erfindung wird durch die nachfolgenden Beispiele verdeutlicht ohne sie darauf zu beschränken.

### Beispiel 1

Direktaminierung von Benzol bie 300 bis 350°C mit in-situ Abtrennung von H₂ direkt aus der Reaktionszone, Pt-GDE/ELAT als Anodenkatalysator

In einer Elektrozelle werden bei einer Temperatur von 300 bis 350°C Benzol und Ammoniak (Eduktstrom E) eingetragen. Die Zelle enthält eine gasdichte MEA mit ausreichend aktiver Fläche. Als protonenleitende Membran der MEA wird Ammoniumpolyphosphat verwendet. Als Elektroden der MEA werden sowohl für Anode als auch für Kathode Gasdiffusionselektroden ELAT (1 mg/cm² Platin) der BASF Fuel Cell GmbH eingesetzt. Außerdem wird an der Retentatseite der Elektrozelle ein Aminierungskatalysator aus einem Kohlenstoffgewebe enthaltend feindispergierte NiCu-Legierung (ca. 3 bis 5 mg/cm²) eingebaut, diese Katalysatorschicht wird direkt an die ELAT Gasdiffusionselektrode angebracht.
1. Aminierungskatalysator bestehend aus einem Kohlenstoffgewebe enthaltend feindispergierte NiCu-Legierung (3 bis 5 mg/cm²),
2. ELAT Gasdiffusionselektrode,
3. protonenleitende Membran (Ammoniumpolyphosphat) und
4. ELAT Gasdiffusionselektrode.

Das Reaktionsgemisch R enthält Benzol, Anilin, Wasserstoff, Stickstoff und Ammoniak und wird auf der Anodenseite der Elektrozelle entlanggeführt. Sauerstoff (oder Luft) wird auf der Kathodenseite in die Elektrozelle eingeleitet. Auf der Retentatseite wird der Wasserstoff am Anodenkatalysator zu Protonen oxidiert, diese durchqueren die Membran und reagieren auf der Permeatseite am Kathodenkatalysator mit dem Sauerstoff zu Wasser. Die treibende Kraft ist die Reduktion des Sauerstoffs. Bei der Gesamtreaktion wird Energie in Form von Wärme und durch Zwischenschalten eines Verbrauchers in Form von elektrischem Strom frei.

Als Reaktoraustrag auf der Anodenseite erhält man ein Gemisch aus Benzol, Anilin, Wasserstoff, Stickstoff und Ammoniak. Ein Großteil des Wasserstoffs wird mittels der Elektromembrane vom Reaktionsgemisch R direkt aus der Reaktionszone abgetrennt und somit wird der Gleichgewichtsumsatz von Benzol zum Anilin erhöht.

### Beispiel 2:

Direktaminierung von Benzol mit in-situ Abtrennung von H₂ bei 300-350°C direkt aus der Reaktionszone, Pd-Folie als Anodenkatalysator

In einer Elektrozelle werden bei einer Temperatur von 300 bis 350°C Benzol und Ammoniak (Eduktstrom E) eingetragen. Die Zelle enthält eine gasdichte MEA mit ausreichend aktiver Fläche. Als protonenleitende Membran der MEA wird Ammoniumpolyphosphat verwendet. Als Anodenelektrode der MEA wird Palladium Folie (Hersteller Goodfellow, Dicke 10 µm) eingesetzt, als Kathode wird eine Gasdiffusionselektrode ELAT mit Pt Beladung von 1 mg/cm² eingesetzt (Hersteller BASF Fuel Cell GmbH). Der Vorteil von Pd Folie ist, dass diese gasdicht ist und damit einen Schutz der keramischen Membrane vor Ammoniak bietet. Außerdem wird an der Retentatseite der Elektrozelle ein Aminierungskatalysator aus einem Kohlenstoffgewebe enthaltend feindispergierte NiCu Legierung (ca. 3-5 mg/cm²) eingebaut, diese Katalysatorschicht wurde direkt an die Pd Folie angebracht.

Die MEA hat demnach einen Schichtaufbau mit - beginnend auf der Retentatseite-folgender Schichtreihenfolge:
1. Aminierungskatalysator bestehend aus einem Kohlenstoffgewebe enthaltend feindispergierte NiCu-Legierung (ca. 3 bis 5 mg/cm³),
2. Palladium Folie (Hersteller Goodfellow, Dicke 10 µm),
3. protonenleitende Membran (Ammoniumpolyphosphat) und
4. Gasdiffusionselektrode ELAT mit Pt Beladung von 1 mg/cm².

Das Reaktionsgemisch R enthält Benzol, Anilin, Wasserstoff, Stickstoff und Ammoniak und wird auf der Anodenseite in die Elektrozelle eingeleitet. Sauerstoff (oder Luft) wird auf der Kathodenseite der Elektrozelle entlanggeleitet. Auf der Retentatseite wird der Wasserstoff am Anodenkatalysator zu Protonen oxidiert, diese durchqueren die Membran und reagieren auf der Permeatseite am Kathodenkatalysator mit dem Sauerstoff zu Wasser. Die treibende Kraft ist die Reduktion des Sauerstoffs. Bei der Gesamtreaktion wird Energie in Form von Wärme und durch Zwischenschalten eines Verbrauchers in Form von elektrischem Strom frei.

Als Reaktoraustrag auf der Anodenseite erhält man ein Gemisch aus Benzol, Anilin, Wasserstoff, Stickstoff und Ammoniak. Ein Großteil des Wasserstoffs wird mittels der Elektromembrane vom Reaktionsgemisch R direkt aus der Reaktionszone abgetrennt und somit wird der Gleichgewichtsumsatz von Benzol zum Anilin erhöht.

### Beispiel 3

In einer Elektrozelle, die eine gasdichte MEA mit 45 cm² aktiver Fläche enthält, wurden 80 l/h Stickstoff und 0,5 l/h Wasserstoff (Wasserstoffkonzentration 6200 ppm) eingetragen. Als Membran der MEA wird CeltecP^{®} (Polyimidazol/Phosphorsäure) der BASF Fuel Cell GmbH verwendet. Als Elektroden der MEA werden sowohl für Anode als auch für Kathode Gasdiffussionselektroden ELAT (1 mg/cm² Platin) der BASF Fuel Cell GmbH eingesetzt. Die Elektrozelle wurde bei 190°C und 1 bar Druck betrieben. An der Anode der MEA lag eine Spannung (U_{A}) von +250 mV an.

Im Austrag der Elektrozelle auf der Anodenseite waren 40 ppm Wasserstoff und Stickstoff (Rest) vorhanden. Dies entspricht einer Wasserstoffabtrennung von 99,4 %.

### Beispiel 4

In Tabelle 1 wird die Effektivität der Wasserstoffabtrennung aus der Beispiel 3 mit Vergleichsbeispielen aus dem Stand der Technik verglichen.

**Tabelle 1:**

| Beispiel | Material MEA | Temperatur | Spannung | Wasserstoffkonzentation | | Druckunterschied Retentat-/Permeatseite | | H₂ Permeance |
|---|---|---|---|---|---|---|---|---|
| | | °C | mV | Eintrag | Austrag | bar | Pa | mol/m².s.Pa |
| Beispiel #3 | Pt-GDE/Celtec P | 190 | 250 | 6200 ppm | 40 ppm | 0,00062 | 62 | 1,99E-05 |
| Vergleichsbeispiel 2 | Sol-Gel Si/AlOₓ | 200 | | | | | | 5,00E-07 |
| Vergleichsbeispiel 3 | Pd-Ag/a-Al₂O₃ | 200-340 | | | | 0,8-2,5 | 100000 | 1,40E-06 |
| Vergleichsbeispiel 4 | Pd | 200 | | | | 0,51 | 51000 | 5,20E-07 |

| | | | | | | | | |
|---|---|---|---|---|---|---|---|---|
| *Vergleichsbeispiel 2 Reference* # 55 *in* Ind. Eng. Chem. Res. 2006, 45, 875-881; *Vergleichsbeispiel 3 Reference* # *189 in* Top. Catal. (2008), 51: 107-122*;* *Vergleichsbeispiel 4 Reference* # *158 in* Top. Catal. (2008), 51: 107-122*.* | | | | | | | | |

## Patentansprüche

1. Verfahren zur Direktaminierung von Kohlenwasserstoffen zu Aminokohlenwasserstoffen durch Umsetzen eines Eduktstroms E, der mindestens einen Kohlenwasserstoff und mindestens ein Aminierungsreagenz enthält, zu einem Reaktionsgemisch R, enthaltend Aminokohlenwasserstoff und Wasserstoff in einer Reaktionszone RZ, und elektrochemische Abtrennung mindestens eines Teils des bei der Umsetzung entstandenen Wasserstoffs aus dem Reaktionsgemisch R mittels einer gasdichten Membran-Elektroden-Assembly, die mindestens eine selektiv protonenleitende Membran und auf jeder Seite der Membran mindestens einen Elektrodenkatalysator aufweist, wobei auf der Retentatseite der Membran mindestens ein Teil des Wasserstoffs an dem Anodenkatalysator zu Protonen oxidiert wird und die Protonen nach Durchqueren der Membran auf der Permeatseite an dem Kathodenkatalysator mit Sauerstoff zu Wasser umgesetzt werden, wobei der Sauerstoff aus einem Sauerstoff enthaltenden Strom O stammt, der mit der Permeatseite der Membran in Kontakt gebracht wird.

2. Verfahren gemäß Anspruch 1, **dadurch gekennzeichnet, dass** der Wasserstoff aus dem Reaktionsgemisch R direkt aus der Reaktionszone RZ abgetrennt wird.

3. Verfahren gemäß Anspruch 1 oder 2, **dadurch gekennzeichnet, dass** aus dem Reaktionsgemisch R mindestens 30 %, bevorzugt mindestens 50 %, besonders bevorzugt mindestens 70 %, ganz besonders bevorzugt mindestens 95 % und insbesondere bevorzugt mindestens 98 % des im Reaktionsgemisch R enthaltenen Wasserstoffs abgetrennt werden.

4. Verfahren gemäß einem der Ansprüche 1 bis 3, **dadurch gekennzeichnet, dass** die Wasserstoffabtrennung bei Temperaturen von 20 bis 800 °C, bevorzugt bei 50 bis 700, und insbesondere bevorzugt von 70 bis 350 °C durchgeführt wird.

5. Verfahren gemäß einem der Ansprüche 1 bis 4, **dadurch gekennzeichnet, dass** der Sauerstoff enthaltende Strom mindestens 15 mol-% Sauerstoff enthält.

6. Verfahren gemäß einem der Ansprüche 1 bis 5, **dadurch gekennzeichnet, dass** die Elektroden der Membran-Elektroden-Assembly als Gasdiffusionselektroden ausgestaltet sind.

7. Verfahren gemäß einem der Ansprüche 1 bis 6, **dadurch gekennzeichnet, dass** die Elektroden der Membran-Elektroden-Assembly als metallische Folien ausgestaltet sind.

8. Verfahren gemäß einem der Ansprüche 1 bis 7, **dadurch gekennzeichnet, dass** als selektiv protonenleitende Membran Membranen ausgewählt aus der Gruppe Keramikmembranen und Polymembranen eingesetzt werden.

9. Verfahren gemäß einem der Ansprüche 1 bis 8, **dadurch gekennzeichnet, dass** der Anodenkatalysator auf der Retentatseite gleichzeitig der Aminierungskatalysator für die Umsetzung der Kohlenwasserstoffe für die Direktaminierung von Kohlenwasserstoffen zu Aminokohlenwasserstoffen ist.

10. Verfahren gemäß einem der Ansprüche 1 bis 9, **dadurch gekennzeichnet, dass** nach Abtrennung mindestens eines Teils des Wasserstoffs ein Produktstrom P erhalten wird, aus dem Aminokohlenwasserstoff und Aminierungsreagenz abgetrennt werden, wobei ein aufgearbeiteter Strom S1 entsteht, der erneut in die Direktaminierung eingesetzt wird.

11. Verfahren gemäß einem der Ansprüche 1 bis 9, **dadurch gekennzeichnet, dass** nach Abtrennung mindestens eines Teils des Wasserstoffs ein Produktstrom P erhalten wird, aus dem Aminokohlenwasserstoff abgetrennt wird, wobei ein aufgearbeiteter Strom S2 entsteht, der erneut in die Direktaminierung eingesetzt wird.

12. Verfahren gemäß einem der Ansprüche 1 bis 9, **dadurch gekennzeichnet, dass** nach Abtrennung mindestens eines Teils des Wasserstoffs ein Produktstrom P erhalten wird, der erneut in die Direktaminierung eingesetzt wird.

13. Verfahren gemäß einem der Ansprüche 1 bis 12, **dadurch gekennzeichnet, dass** als Kohlenwasserstoff Toluol und als Aminierungsreagenz Ammoniak eingesetzt wird, und als Aminokohlenwasserstoff Toluoldiamin entsteht.

14. Verfahren gemäß einem der Ansprüche 1 bis 13, **dadurch gekennzeichnet, dass** als Kohlenwasserstoff Benzol und als Aminierungsreagenz Ammoniak eingesetzt wird, und als Aminokohlenwasserstoff Anilin entsteht.

15. Verfahren gemäß einem der Ansprüche 1 bis 14, **dadurch gekennzeichnet, dass** als Kohlenwasserstoff Methan und als Aminierungsreagenz Ammoniak eingesetzt wird, und als Aminokohlenwasserstoff mindestens ein Amin aus der Gruppe Methylamin, Dimethylamin und Trimethylamin entsteht.

## Claims

1. A process for the direct amination of hydrocarbons to aminohydrocarbons by reaction of a feed stream E comprising at least one hydrocarbon and at least one aminating reagent to form a reaction mixture R comprising aminohydrocarbon and hydrogen in a reaction zone RZ and electrochemical separation of at least part of the hydrogen formed in the reaction from the reaction mixture R by means of a gastight membrane-electrode assembly having at least one selectively proton-conducting membrane and at least one electrode catalyst on each side of the membrane, where at least part of the hydrogen is oxidized to protons at the anode catalyst on the retentate side of the membrane and the protons pass through the membrane and on the permeate side are reacted with oxygen to form water, where the oxygen originates from an oxygen-comprising stream 0 which is brought into contact with the permeate side of the membrane, over the cathode catalyst.

2. The process according to claim 1, wherein the hydrogen is separated off from the reaction mixture R directly from the reaction zone RZ.

3. The process according to claim 1 or 2, wherein, at least 30%, preferably at least 50%, particularly preferably at least 70%, very particularly preferably at 95% and in particular at least 98%, of the hydrogen comprised in the reaction mix R is separated off from the reaction mixture R.

4. The process according to any of claims 1 to 3, wherein the hydrogen removal is carried out at temperatures of from 20 to 800°C, preferably from 50 to 700°C and in particular from 70 to 350°C.

5. The process according to any of claims 1 to 4, wherein the oxygen-comprising stream comprises at least 15 mol% of oxygen.

6. The process according to any of claims 1 to 5, wherein the electrodes of the membrane-electrode assembly are configured as gas diffusion electrodes.

7. The process according to any of claims 1 to 6, wherein the electrodes of the membrane-electrode assembly are configured as metallic foils.

8. The process according to any of claims 1 to 7, wherein membranes selected from the group consisting of ceramic membranes and polymer membranes are used as selectively proton-conducting membrane.

9. The process according to any of claims 1 to 8, wherein the anode catalyst on the retentate side is simultaneously the amination catalyst for the reaction of the hydrocarbons in the direct amination of hydrocarbons to aminohydrocarbons.

10. The process according to any of claims 1 to 9, wherein removal of at least part of the hydrogen gives a product stream P from which aminohydrocarbon and aminating reagent are separated off to give a worked-up stream S1 which is reused in the direct amination.

11. The process according to any of claims 1 to 9, wherein removal of at least part of the hydrogen gives a product stream P from which aminohydrocarbon is separated off to give a worked-up stream S2 which is reused in the direct amination.

12. The process according to any of claims 1 to 9, wherein removal of at least part of the hydrogen gives a product stream P which is reused in the direct amination.

13. The process according to any of claims 1 to 12, wherein toluene is used as hydrocarbon and ammonia is used as aminating reagent and toluenediamine is formed as aminohydrocarbon.

14. The process according to any of claims 1 to 13, wherein benzene is used as hydrocarbon and ammonia is used as aminating reagent and aniline is formed as aminohydrocarbon.

15. The process according to any of claims 1 to 14, wherein methane is used as hydrocarbon and ammonia is used as aminating reagent and at least one amine from the group consisting of methylamine, dimethylamine and trimethylamine is formed as aminohydrocarbon.

## Revendications

1. Procédé pour l'amination directe d'hydrocarbures en aminohydrocarbures par transformation d'un flux de départ E, qui contient au moins un hydrocarbure et au moins un réactif d'amination, en un mélange réactionnel R, contenant l'aminohydrocarbure et de l'hydrogène dans une zone de réaction RZ, et par séparation électrochimique d'au moins une partie de l'hydrogène formé lors de la transformation du mélange réactionnel R au moyen d'un ensemble d'électrode à membrane imperméable aux gaz, qui présente au moins une membrane guidant les protons de manière sélective et, sur chaque côté de la membrane, au moins un catalyseur à électrode, au moins une partie de l'hydrogène étant oxydée, sur le côté retentat de la membrane, sur le catalyseur anodique, en protons et les protons étant transformés, après passage au travers de la membrane, sur le côté perméat, sur le catalyseur cathodique, avec de l'oxygène en eau, l'oxygène provenant d'un flux 0 contenant de l'oxygène qui est mis en contact avec le côté perméat de la membrane.

2. Procédé selon la revendication 1, **caractérisé en ce que** l'hydrogène est séparé du mélange réactionnel R directement de la zone de réaction RZ.

3. Procédé selon la revendication 1 ou 2, **caractérisé en ce qu'**on sépare du mélange réactionnel R au moins 30%, de préférence au moins 50%, de manière particulièrement préférée au moins 70%, de manière tout particulièrement préférée au moins 95% et en particulier de préférence au moins 98% de l'hydrogène contenu dans le mélange réactionnel R.

4. Procédé selon l'une quelconque des revendications 1 à 3, **caractérisé en ce que** la séparation de l'hydrogène est réalisée à des températures de 20 à 800°C, de préférence de 50 à 700°C, en particulier de préférence de 70 à 350°C.

5. Procédé selon l'une quelconque des revendications 1 à 4, **caractérisé en ce que** le flux contenant de l'oxygène contient au moins 15% en mole d'oxygène.

6. Procédé selon l'une quelconque des revendications 1 à 5, **caractérisé en ce que** les électrodes de l'ensemble d'électrode à membrane sont conçues comme électrodes à diffusion de gaz.

7. Procédé selon l'une quelconque des revendications 1 à 6, **caractérisé en ce que** les électrodes de l'ensemble d'électrode à membrane sont conçues comme des feuilles métalliques.

8. Procédé selon l'une quelconque des revendications 1 à 7, **caractérisé en ce qu'**on utilise, comme membrane guidant les protons de manière sélective, des membranes choisies dans le groupe formé par les membranes céramiques et les membranes polymères.

9. Procédé selon l'une quelconque des revendications 1 à 8, **caractérisé en ce que** le catalyseur anodique sur le côté retentat est simultanément le catalyseur d'amination pour la transformation des hydrocarbures et pour l'amination directe d'hydrocarbures en aminohydrocarbures.

10. Procédé selon l'une quelconque des revendications 1 à 9, **caractérisé en ce qu'**on obtient, après la séparation d'au moins une partie de l'hydrogène, un flux de produits P dont on sépare l'aminohydrocarbure et le réactif d'amination, avec formation d'un flux S1 traité, qui est à nouveau utilisé dans l'amination directe.

11. Procédé selon l'une quelconque des revendications 1 à 9, **caractérisé en ce qu'**on obtient, après la séparation d'au moins une partie de l'hydrogène, un flux de produits P dont on sépare l'aminohydrocarbure, avec formation d'un flux S2 traité, qui est à nouveau utilisé dans l'amination directe.

12. Procédé selon l'une quelconque des revendications 1 à 9, **caractérisé en ce qu'**on obtient, après la séparation d'au moins une partie de l'hydrogène, un flux de produits P, qui est à nouveau utilisé dans l'amination directe.

13. Procédé selon l'une quelconque des revendications 1 à 12, **caractérisé en ce qu'**on utilise, comme hydrocarbure, du toluène et, comme réactif d'amination, de l'ammoniaque et on obtient, comme aminohydrocarbure, de la toluènediamine.

14. Procédé selon l'une quelconque des revendications 1 à 13, **caractérisé en ce qu'**on utilise, comme hydrocarbure, du benzène et, comme réactif d'amination, de l'ammoniaque et on obtient, comme aminohydrocarbure, de l'aniline.

15. Procédé selon l'une quelconque des revendications 1 à 14, **caractérisé en ce qu'**on utilise, comme hydrocarbure, du méthane et, comme réactif d'amination, de l'ammoniaque et on obtient, comme aminohydrocarbure, au moins une amine du groupe formé par la méthylamine, la diméthylamine et la triméthylamine.
